# EUROPÄISCHE PATENTSCHRIFT

(11) **EP 1 167 311 B1**
(45) Veröffentlichungstag und Bekanntmachung des Hinweises auf die Patenterteilung: **14.04.2004**
(21) Anmeldenummer: 01114794.9
(22) Anmeldetag: 27.06.2001
(51) Int. Cl.: C03C 10/16, A61K 6/06

(54) **Tiefsinternde Apatit-Glaskeramik**
Low sintering apatite glass ceramics
Vitrocéramique en apatite à basse température de frittage

(30) Priorität: 28.06.2000 DE 10031430
(43) Veröffentlichungstag der Anmeldung: 02.01.2002
(73) Patentinhaber: Ivoclar Vivadent AG, 9494 Schaan (LI)
(72) Erfinder: Schweiger, Marcel, 7000 Chur (CH); Rheinberger, Volker, 9490 Vaduz (LI); Höland, Wolfram, 9494 Schaan (LI)
(74) Vertreter: UEXKÜLL & STOLBERG

(56) Entgegenhaltungen:
- EP-A- 0 885 855
- EP-A- 0 885 856

## Beschreibung

Die Erfindung betrifft eine tiefsinternde Apatit-Glaskeramik, die sich insbesondere zum Einsatz in der restaurativen Zahnheilkunde und vor allem zur Beschichtung oder Verblendung von dentalen Restaurationen, wie Schalen, Veneers, Brücken oder Kronen, eignet.

Glaskeramiken für die Verwendung im Dentalbereich sind aus dem Stand der Technik bekannt.

Die EP-A-0 690 030 offenbart leucithaltige Phosphosilicat-Glaskeramiken, die in der Dentaltechnik eingesetzt werden können. Aufgrund des Leucit-Gehaltes haben sie allerdings sehr hohe lineare thermische Ausdehnungskoeffizienten, so daß sie für die Beschichtung von Werkstoffen mit niedrigen Ausdehnungskoeffizienten, wie z.B. Lithiumdisilicat-Glaskeramiken, nicht geeignet sind.

Weiter sind in der EP-A-0 695 726 Alkali-Zink-Silicat-Glaskeramiken offenbart, die jedoch nur maximal 8,0 Gew.-% ZnO enthalten können, weshalb deren chemische Beständigkeit noch nicht in jedem Falle zufriedenstellend ist. Diese Glaskeramiken besitzen überdies den Nachteil, daß sie keinen Apatit, sondern als Kristallphase Leucit enthalten. Infolge des hohen Ausdehnungskoeffizienten von Leucit eignen sich die Glaskeramiken daher in der Regel ebenfalls nicht als Beschichtungen für Lithiumdisilicat-Glaskeramiken.

Auch Apatit-Glaskeramiken sind bereits in der restaurativen Zahnheilkunde verwendet worden.

Die EP-A-885 855 und EP-A-885 856 beschreiben Apatit-Glaskeramiken mit optischen Eigenschaften, die denen des natürlichen Zahnes nahekommen. Sie zeigen eine gute Beständigkeit unter den Bedingungen des Mundmilieus und leiten sich aus dem chemischen System SiO₂-Al₂O₃-P₂O₅-K₂O-Na₂O-CaO-F ab. Es sind Zusatzkomponenten möglich, aber nur in verhältnismäßig geringen Mengen. So ist der Gehalt an ZnO auf maximal 5,0 Gew.-% und der von K₂O auf maximal 8,5 Gew.-% beschränkt. Infolge dieser Beschränkungen ist mit diesen Materialien noch nicht in jedem Fall eine Kombination von guter chemischer Beständigkeit und niedriger Sintertemperatur erzielbar.

Ein weiterer Nachteil dieser Glaskeramiken besteht darin, daß sie in der Regel nicht bei niedrigen Temperaturen von weniger als 800°C auf ein keramisches oder glaskeramisches Dentalgerüst, wie eine Lithiumdisilikat-Glaskeramik, aufgesintert werden können. Durch die erforderlichen hohen Sintertemperaturen kommt es aber gerade bei der Herstellung von dünnwandigen Dentalrestaurationen, wie Verblendschalen, zu Spannungen und zum Bruch der Dentalrestauration. Somit sind insbesondere Dentalschalen mit einem Kern aus Lithiumdisilikat-Glaskeramik und darauf geschichteter Apatit-Glaskeramik nach dem Stand der Technik nicht in befriedigender Weise herstellbar.

Weiter ist die zufriedenstellende Verarbeitung der bekannten Glaskeramiken durch Sinterung nur in einem engen Temperaturbereich möglich. Bei größeren Abweichungen von der eigentlichen Sintertemperatur zeigen diese Glaskeramiken im Falle zu hoher Temperatur eine unbefriedigende Formstabilität und im Falle zu niedriger Temperatur eine inakzeptabel hohe Porosität nach der Sinterung. Die zufriedenstellende Verarbeitbarkeit nur in einem engen Temperaturintervall ist sehr nachteilig, da gerade die für die Herstellung von Dentalrestaurationen eingesetzten Öfen klein sind, und es somit generell bei ihnen schwierig ist, eine gewünschte Temperatur über einen gewissen Zeitraum konstant aufrechtzuerhalten. Besonders bei Öfen, die bei niedrigen Temperaturen, wie kleiner als 850°C, arbeiten, treten während eines Sintervorganges erhebliche Temperaturschwankungen auf.

Der Erfindung liegt demgemäß die Aufgabe zugrunde, eine Apatit-Glaskeramik zur Verfügung zu stellen, die in ihren optischen Eigenschaften und insbesondere ihrer hohen Transluzenz natürlichem Zahnmaterial ähnelt und dabei eine ausgezeichnete chemische Beständigkeit und einen geringen thermischen Ausdehnungskoeffizienten aufweist. Weiterhin soll die Apatit-Glaskeramik eine niedrige Sintertemperatur haben, so dass sie sich vor allem als Beschichtungs- oder Verblendmaterial zur Herstellung von stabilen dünnwandigen dentalen Restaurationen, wie Dentalschalen, eignet. Schließlich soll die Glaskeramik in einem breiten Temperaturbereich zu den gewünschten Restaurationen verarbeitbar sein.

Diese Aufgabe wird überraschenderweise durch die tiefsinternde Apatit-Glaskeramik nach den Ansprüchen 1 bis 9 gelöst.

Gegenstand der Erfindung sind ebenfalls das Verfahren zur Herstellung der Apatit-Glaskeramik nach Anspruch 10, das Dentalmaterial nach den Ansprüchen 11 bis 14, die Verwendung nach den Ansprüchen 15 bis 18 sowie die geformten Dentalprodukte nach den Ansprüchen 19 bis 22.

Die erfindungsgemäße Apatit-Glaskeramik ist dadurch gekennzeichnet, daß sie die folgenden Komponenten enthält.

| Komponente | Gew.-% |
|---|---|
| SiO₂ | 56,0 bis 65,0 |
| Li₂O | 1,8 bis 5,3 |
| K₂O | 9,0 bis 17,5 |
| ZnO | 8,9 bis 16,0 |
| CaO | 3,5 bis 10,5 |
| P₂O₅ | 2,0 bis 6,0 |
| F | 0,4 bis 1,0. |

und die Hauptkristallphase durch Apatit-Kristalle gebildet ist.

Die erfindungsgemäße Glaskeramik kann zusätzlich noch mindestens eine der folgenden Komponenten enthalten:

| Komponente | Gew.-% |
|---|---|
| Na₂O | 0 bis 5, 0 |
| MgO | 0 bis 3,5 |
| SrO | 0 bis 3,5 |
| Al₂O₃ | 0 bis 6,0 |
| B₂O₃ | 0 bis 2 , 0 |
| La₂O₃ | 0 bis 3,0 |
| ZrO₂ | 0 bis 7,5 |
| TiO₂ | 0 bis 7,5 |
| CeO₂ | 0 bis 2,0 |
| SnO₂ | 0 bis 5,0 |
| Tb₄O₇ | 0 bis 0,5. |

Sofern diese zusätzlichen Komponenten vorhanden sind, werden sie insbesondere in Mengen von mindestens 0,1 Gew.-% eingesetzt.

Für die einzelnen Komponenten der erfindungsgemäßen Apatit-Glaskeramik existieren bevorzugte Mengenbereiche. Diese können, sofern nicht anders angegeben, unabhängig voneinander gewählt werden und sind wie folgt:

| Komponente | Gew.-% |
|---|---|
| SiO₂ | 56,0 bis 64,0 |
| Li₂O | 2,0 bis 5,0 |
| K₂O | 9,5 bis 16,0 |
| ZnO | 8,9 bis 15,0 |
| CaO | 4,0 bis 10,0 |
| P₂O₅ | 2,0 bis 5,0 |
| F | 0,4 bis 0,9 |
| Na₂O | 0 bis 4 , 0 |
| MgO | 0 bis 3,0 |
| SrO | 0 bis 3,0 |
| Al₂O₃ | 0 bis 5,0 |
| B₂O₃ | 0 bis 1,8 |
| La₂O₃ | 0 bis 2,5 |
| ZrO₂ | 0 bis 6,0 |
| TiO₂ | 0 bis 6,0 |
| CeO₂ | 0 bis 1,8 |
| SnO₂ | 0 bis 4,0 |
| Tb₄O₇ | 0 bis 0, 4 . |

Besonders bevorzugte Mengenbereiche für die einzelnen Komponenten der erfindungsgemäßen Apatit-Glaskeramik sind wie folgt und diese können unabhängig voneinander gewählt werden:

| Komponente | Gew.-% |
|---|---|
| SiO₂ | 56,0 bis 63,0 |
| Li₂O | 2,5 bis 5,0 |
| K₂O | 10,0 bis 15,0 |
| ZnO | 8,9 bis 14,0 |
| CaO | 4,0 bis 9,0 |
| P₂O₅ | 2,5 bis 5,0 |
| F | 0,4 bis 0,8 |
| Na₂O | 0 bis 3,0 |
| MgO | 0 bis 2,5 |
| SrO | 0 bis 2,5 |
| Al₂O₃ | 0 bis 4,0 |
| B₂O₃ | 0 bis 1,5 |
| La₂O₃ | 0 bis 2,0 |
| ZrO₂ | 0 bis 5,0 |
| TiO₂ | 0 bis 5,0 |
| CeO₂ | 0 bis 1,5 |
| SnO₂ | 0 bis 3,0 |
| Tb₄O₇ | 0 bis 0,3. |

Alle vorstehenden Mengen in Gew.-% beziehen sich auf die Glaskeramik.

Die erfindungsgemäße Glaskeramik kann weiter z.B. übliche Farbkomponenten zur Anpassung an die Farbe des natürlichen Zahnmaterials eines Patienten enthalten.

Durch Rasterelektronenmikroskop- und Röntgenbeugungsuntersuchungen konnte festgestellt werden, daß in der Glaskeramik Apatit, wie Hydroxy- und/oder Fluor-Apatit, die Hauptkristallphase bildet. Die Apatitkristalle sind bevorzugt hexagonal und insbesondere nadelförmig gewachsen. Die Apatitkristalle sind in ihrer größten Ausdehnung bevorzugt kleiner als 10 µm, insbesondere kleiner als 7 µm und besonders bevorzugt kleiner als 5 µm.

Durch die ausgeschiedenen Apatitkristalle, die in ihrem Aussehen den Carbonat-Apatit-Kristallen des natürlichen Zahnmaterials ähneln, werden die optischen Eigenschaften der Glaskeramik gesteuert. So ist es möglich, daß eine Glaskeramik mit einem Aussehen erzeugt wird, welches dem des Dentins oder Schmelzes des Zahnes entspricht. Gleichzeitig wird eine optische Tiefenwirkung in der Glaskeramik erzielt, wie es durch andere Kristall-Typen nicht möglich ist.

Bei der erfindungsgemäßen Glaskeramik sind röntgenographisch keine Leucitkristalle nachweisbar, allerdings können Nebenkristallphasen, wie z.B. Natrium-Calcium-Orthophosphat vom Typ NaCaPO₄ vorhanden sein.

Ein weiterer besonderer Vorteil der erfindungsgemäßen Glaskeramik besteht darin, daß sie aufgrund ihrer besonderen Zusammensetzung nicht nur eine hohe chemische Beständigkeit und Transluzenz, sondern auch eine besonders erwünschte niedrige Sintertemperatur besitzt.

Die erfindungsgemäße Glas hat üblicherweise beim Aufsintern auf ein keramisches oder glaskeramisches Substrat, wie eine Lithiumdisilikat-Glaskeramik eine sehr vorteilhafte Sintertemperatur von weniger als 800°C. Besonders bevorzugt sind solche Glaskeramiken, die ein Sintertemperatur von 780°C und weniger haben und damit bei dieser Temperatur verarbeitet werden können. Diese niedrigen Sintertemperaturen sind vermutlich auf die spezielle Zusammensetzung der erfindungsgemäßen Glaskeramik zurückzuführen.

Es ist von ganz besonderem Vorteil, dass die erfindungsgemäße Glaskeramik auch noch bei großen Abweichungen von der eigentlichen Sintertemperatur, d. h. der Temperatur, bei der die Formstabilität sowie die Porosität des Glaskeramik besonders zufriedenstellend sind, durch Sinterung verarbeitet werden kann. So kann die Glaskeramik sogar in einem Sintertemperaturbereich von ± 20°C, oder mehr, wie z.B. ± 40°C, ober- bzw. unterhalb der eigentlichen Sintertemperatur verarbeitet werden, ohne dass Risse oder Sprünge in der Dentalrestauration auftreten. Bei einer Verarbeitung in diesem Temperaturbereich weist die gesinterte Glaskeramik eine sehr geringe Porosität und eine sehr gute Formstabilität auf. Die ausgezeichnete Formstabilität zeigt sich daran, dass selbst die sehr dünnwandige Zahnschneidekante, die durch Aufbringen eines Gemisches aus Glaskeramikpulver und Anmischflüssigkeit auf ein Gerüst sowie dessen Formung gebildet wurde, nach dem Sintervorgang ihre Form behält und damit bestehen bleibt. Damit kann die erfindungsgemäße Glaskeramik auch in solchen Öfen gesintert werden, die eine genaue Konstanthaltung der Brenntemperatur nicht gestatten, was ein besonderer Vorteil ist. Demgegenüber gestatten konventionelle Glaskeramiken lediglich Abweichungen von ± 10°C von der Sintertemperatur. Bei größeren Abweichungen sind mit ihnen zufriedenstellende Restaurationen nicht herstellbar.

Weiter hat die Apatit-Glaskeramik üblicherweise einen niedrigen thermischen Ausdehnungskoeffizienten von 9,3 bis 10,8 × 10⁻⁶K⁻¹, gemessen im Temperaturbereich von 100 °C bis 400 °C.

Zur Herstellung der erfindungsgemäßen Apatit-Glaskeramik wird
a) ein Ausgangsglas, welches die oben angegebenen Komponenten enthält, bei Temperaturen von 1200°C bis 1650°C erschmolzen,
b) die erhaltene Glasschmelze unter Bildung eines Glasgranulates in Wasser eingegossen,
c) das Glasgranulat gegebenenfalls zu einem Glaspulver mit einer mittleren Korngröße von 1 bis 450 µm, bezogen auf die Teilchenzahl, zerkleinert, und
d) das Glasgranulat oder das Glaspulver einer thermischen Behandlung bei mehr als 500°C und bis zu 900°C für eine Dauer von 30 Minuten bis 6 Stunden unterzogen.

In Stufe (a) wird zunächst ein Ausgangsglas erschmolzen, indem geeignete Ausgangsmaterialien, wie zum Beispiel Carbonate, Oxide und Fluoride, innig miteinander vermischt und auf die angegebenen Temperaturen erwärmt werden.

Dann wird in Stufe (b) die erhaltene Glasschmelze durch Eingießen in Wasser abgeschreckt und dadurch zu einem Glasgranulat umgewandelt. Diese Vorgehensweise wird üblicherweise auch als Fritten bezeichnet.

Gegebenenfalls wird das Glasgranulat anschließend in Stufe (c) zerkleinert und insbesondere mit üblichen Mühlen auf die gewünschte Korngröße gemahlen. Dabei hat das erhaltene Glaspulver bevorzugt eine mittlere Korngröße von 1 bis 450 µm, bezogen auf die Teilchenzahl.

In Stufe (d) wird das Glasgranulat oder gegebenenfalls das Glaspulver einer thermischen Behandlung bei einer Temperatur im Bereich von mehr als 500°C und bis zu 900°C für eine Dauer von 30 Minuten bis 6 Stunden, vorzugsweise 30 Minuten bis 3 Stunden, unterzogen. Im Gegensatz zu den konventionellen Apatit-Glaskeramiken ist es möglich, die Temperaturbehandlung und damit die Erzeugung der Apatit-Kristalle bei Temperaturen von weniger als 900°C durchzuführen, was fraglos ein Vorteil ist.

Bei der Wärmebehandlung findet eine Volumenkristallisation statt. Diese führt zu einer homogenen Verteilung der Apatitkristalle innerhalb der gesamten Glaskeramik, im Gegensatz zur Leucitkristallisation, die nur an den inneren Oberflächen eines Glaspulvers stattfinden kann.

Durch rasterelektronenmikroskopische und Röntgenbeugungsuntersuchungen konnte festgestellt werden, daß Apatit, vorzugsweise Fluor-Apatit, die Hauptkristallphase bildet. Die Größe der erhalten Kristalle kann dabei durch die gewählte Temperatur und die Dauer der thermischen Behandlung gesteuert werden. Zusätzlich zu den Apatitkristallen können je nach chemischer Zusammensetzung des eingesetzten Ausgangsglases weitere Kristallphasen gebildet werden.

Neben den verschiedenen Kristallphasen können auch mikroheterogene Entmischungsbereiche, d.h. verschiedene Glasphasen vorliegen. Diese Bereiche sind im Rasterelektronenmikroskop als kleine mikroheterogene Tropfenglasphasen mit einer Größe von ca. 20 bis 400 nm erkennbar. Die auftretenden Tropfenglasphasen beeinflussen zusammen mit den Kristallen die optischen Eigenschaften der erfindungsgemäßen Glaskeramiken, wie z.B. Opaleszenz und Transluzenz.

Überraschenderweise können die optischen Eigenschaften der erfindungsgemäßen Apatit-Glaskeramik von glasig transparent bis weißlich trüb eingestellt werden. Dies ist für den Einsatz als Dentalmaterial oder Komponente davon zwingend erforderlich, um alle unterschiedlichen Erscheinungsformen des natürlichen Zahnes reproduzierbar herstellen zu können. Die feinen Apatitkristalle im Gefüge der erfindungsgemäßen Glaskeramik bewirken in Bezug auf Optik und strukturellen Aufbau eine sehr große Ähnlichkeit zum natürlichen Zahn.

Die erfindungsgemäße Apatit-Glaskeramik wird daher entweder alleine oder zusammen mit weiteren Komponenten bevorzugt als Dentalmaterial eingesetzt. Hierzu wird es üblicherweise in Form eines Pulvers mit einer mittleren Teilchengröße von weniger als 90 µm verwendet. Als weitere Komponenten kommen Gläser und andere Glaskeramiken, aber auch Farbstoffe, insbesondere Farbpigmente, Oxide der 3d-Elemente oder Metallkolloide, sowie Fluoreszenzstoffe, insbesondere mit d- und f-Elementen dotiertes Ytterbium-Silicat, in Frage. Dabei ist es bevorzugt, daß das Dentalmaterial 10 bis 90 Gew.-% der Apatit-Glaskeramik enthält.

Bei Einsatz der Apatit-Glaskeramik als Komponente von Dentalmaterial können durch geeignete Wahl ihrer Zusammensetzung sowie der Art der weiteren Komponenten Dentalmaterialien erhalten werden, bei denen wichtige Eigenschaften, wie z.B. Verarbeitungstemperatur, optische Eigenschaften, thermischer Ausdehnungskoeffizient und chemische Beständigkeit, genau an die jeweiligen Anforderungen angepaßt sind. Mit reiner Glaskeramik ist dies häufig nicht möglich.

Besonders vorteilhaft ist Dentalmaterial, das als weitere Komponente mindestens ein Glas und bevorzugt ein Kalium-Zink-Silicat-Glas enthält.

Dabei ist ein Kalium-Zink-Silicat-Glas bevorzugt, das die folgenden Komponenten enthält:

| Komponente | Gew.-% |
|---|---|
| SiO₂ | 60,0 bis 72,0 |
| Li₂O | 1,0 bis 5,0 |
| K₂O | 10,0 bis 23,0 |
| ZnO | 8,5 bis 20,0. |

Dieses Glas kann zusätzlich noch mindestens eine der folgenden Komponenten enthalten:

| Komponente | Gew.-% |
|---|---|
| Na₂O | 0 bis 4,0 |
| MgO | 0 bis 4,0 |
| CaO | 0 bis 3,6 |
| SrO | 0 bis 3,0 |
| Al₂O₃ | 0 bis 8,0 |
| B₂O₃ | 0 bis 3,3 |
| La₂O₃ | 0 bis 3,0 |
| ZrO₂ | 0 bis 6,0 |
| TiO₂ | 0 bis 2,5 |
| CeO₂ | 0 bis 2,0 |
| SnO₂ | 0 bis 5,0 |
| P₂O₅ | 0 bis 1,0 |
| Tb₄O₇ | 0 bis 1,8 |
| F | 0 bis 1,1. |

Sofern diese zusätzlichen Komponenten vorhanden sind, werden sie insbesondere in Mengen von mindestens 0,1 Gew.-% eingesetzt.

Die vorstehende Angaben in Gew.-% beziehen sich auf das Kalium-Zink-Silicat-Glas.

Das Kalium-Zink-Silicat-Glas kann in üblicher Weise hergestellt werden, indem z.B. ein entsprechendes Gemenge von geeigneten Oxiden, Carbonaten und Fluoriden in einem Platin/ Rhodium-Tiegel bei einer Temperatur von 1550°C bis 1600°C während einer Homogenisierungszeit von 1 bis 1,5 Stunden erschmolzen wird. Sofern gewünscht, kann die Glasschmelze dann in Wasser abgeschreckt, und das gebildete Granulat getrocknet und auf eine gewünschte Korngröße aufgemahlen werden.

Das erhaltene Kalium-Zink-Silicat-Glas zeichnet sich durch eine hohe Transluzenz, hohe chemische Beständigkeit sowie einen niedrigen Ausdehnungskoeffizienten aus. Es ist darüberhinaus in seiner chemischen Zusammensetzung ausgezeichnet an die erfindungsgemäße Apatit-Glaskeramik angepaßt, so daß nachteilige Stofftransportreaktionen zwischen beiden Materialien und damit einhergehende Spannungsbildung gerade bei dünnen Schichtverbunden vermieden werden.

Das erfindungsgemäße Dentalmaterial hat normalerweise einen linearen thermischen Ausdehnungskoeffizienten von 9,0 bis 10,9 x 10⁻⁶ K⁻¹, gemessen im Bereich von 100°C bis 400°C. Der jeweils gewünschte Koeffizient kann dabei durch geeignete Wahl der Art der Apatit-Glaskeramik und etwaiger weiterer Komponenten, sowie deren Mengen eingestellt werden. Günstige Dentalmaterialen enthalten 10 bis 90 Gew.-% Apatit-Glaskeramik und 90 bis 10 Gew.-% weitere Komponenten, bezogen auf das Dentalmaterial.

Das erfindungsgemäße Dentalmaterial eignet sich zur Beschichtung von Substraten und insbesondere zur Beschichtung oder Verblendung von dentalen Restaurationen. Die Beschichtung erfolgt dabei insbesondere durch Aufbringen des Dentalmaterials auf das ausgewählte Substrat und anschließendes Sintern bei weniger als 800°C und vorzugsweise bei 760°C oder weniger.

Bevorzugt wird dabei zunächst ein Pulver der erfindungsgemäßen Apatit-Glaskeramik mit einem Pulver der ggf. vorhandenen weitere Komponenten gemischt und durch Zugabe von wäßrigen Anmischlösungen zu einer Paste verarbeitet. Diese Paste wird dann auf das Substrat aufgebracht, und nach gewünschter Formgebung erfolgt das Sintern, um eine fest haftende Beschichtung oder Verblendung zu erhalten.

Das erfindungsgemäße Dentalmaterial kann als Beschichtungs- oder Verblendmaterial für Substrate, wie Dentalsuprastrukturen, z.B. auf Basis von keramischen oder glaskeramischen Werkstoffen eingesetzt werden. Aufgrund seines niedrigen Ausdehnungskoeffizienten wird es bevorzugt bei Substratwerkstoffen mit einem thermischen Ausdehungskoeffizienten von 7,0 bis 12,0, insbesondere von 8,0 bis 11,0 × 10⁻⁶K⁻¹, verwendet werden. Bevorzugt wird es zur Beschichtung oder Verblendung von ZrO₂-Keramiken, Al₂O₃-Keramiken, ZrO₂/Al₂O₃-Keramiken, keramischen oder glaskeramischen Compositwerkstoffen und Titan verwendet.

Besonders vorteilhaft wird es jedoch zur Verblendung von Substraten auf Basis von Lithiumdisilicat-Glaskeramik eingesetzt, um auf diese Weise ästhetisch sehr ansprechende vollkeramische Dentalprodukte herzustellen, die eine sehr hohe Festigkeit sowie eine ausgezeichnete chemische Beständigkeit haben.

Als besonders geeignet haben sich dabei Lithiumdisilicat-Glaskeramiken erwiesen, die die folgenden Komponenten enthalten und z.B. durch Erschmelzen entsprechender Ausgangsgläser, Fritten und Wärmebehandlung bei 400 °C bis 1100 °C erhalten werden können:

| Komponente | Gew.-% |
|---|---|
| SiO₂ | 57,0 bis 80,0 |
| Al₂O₃ | 0 bis 5,0 |
| La₂O₃ | 0,1 bis 6,0 |
| MgO | 0 bis 5,0 |
| ZnO | 0 bis 8,0 |
| Li₂O | 11,0 bis 19,0 |
| P₂O₅ | 0 bis 11,0 |

wobei
(a) Al₂O₃ + La₂O₃ 0,1 bis 7,0 Gew.-% und
(b) MgO + ZnO 0,1 bis 9,0 Gew.-%
ausmachen.

Die Angaben in Gew.-% beziehen sich auf die Lithiumdisilicat-Glaskeramik.

Die erfindungsgemäße Apatit-Glaskeramik und das erfindungsgemäße Dentalmaterial können zusammen mit den ggf. vorhandenen Zusätzen zu geformten Dentalprodukten in üblicher Weise verarbeitet werden. Als erfindungsgemäße geformte Dentalprodukte, die einen Gehalt an der Apatit-Glaskeramik oder dem Dentalmaterial aufweisen, kommen insbesondere Dentalrestaurationen, wie z.B. ein Inlay, ein Onlay, eine Brücke, ein Stiftaufbau, eine Verblendung, eine Facette, eine Füllung oder ein Verbinder in Frage. Besonders bevorzugte Dentalrestaurationen sind Schalen, Veneers, Brücken, Kronen und Teilkronen.

Die Dentalprodukte haben bevorzugt einen Kern auf Basis von keramischem oder glaskeramischem Werkstoff, insbesondere Lithiumdisilicat-Glaskeramik, auf den die erfindungsgemäße Glaskeramik oder das erfindungsgemäße Dentalmaterial aufgebracht ist. Bevorzugte Lithiumdisilicat-Glaskeramiken sind oben bereits beschrieben worden.

Im Gegensatz zu konventionellen Glaskeramiken ist die erfindungsgemäße Glaskeramik in ihrer chemischen Zusammensetzung noch besser an Gläser, wie Kalium-Zink-Silicat-Gläser, und an Lithiumdisilicat-Glaskeramiken angepaßt, die bevorzugt als weitere Komponente eines Beschichtungsmaterials oder als Substrat eingesetzt werden. Das hat zur Folge, daß gerade bei dünnen Schichtverbunden, wie. z.B. Verblendschalen, mit Lithiumdisilicat-Glaskeramik als Substrat, auf das eine Mischung aus erfindungsgemäßer Apatit-Glaskeramik und Kalium-Zink-Silicat-Glas aufgebracht wurde, es nicht zu Ablösungserscheinungen der Beschichtung oder einem Brechen des fertigen Produktes kommt. Für dieses vorteilhafte Verhalten ist auch die niedrige Sintertemperatur der erfindungsgemäßen Glaskeramik verantwortlich.

Überdies zeigt die erfindungsgemäße Glaskeramik eine ausgezeichnete chemische Beständigkeit, die für seine Verwendung als Dentalmaterial unerläßlich ist, welches in der Mundhöhle permanent von sauren Flüssigkeiten umspült wird. Es ist überraschend, daß die Glaskeramik sowohl eine gute chemische Beständigkeit als auch eine niedrige Sintertemperatur aufweist. Möglicherweise ist diese günstige Kombination von Eigenschaften darauf zurückzuführen, daß die Glaskeramik gleichzeitig mehrere Arten von Alkalimetall-Ionen enthält.

Die Erfindung wird nachstehend anhand von Beispielen näher erläutert.

### Beispiele

### Beispiel 1 bis 3 und 5 bis 31

Es wurden insgesamt 30 verschiedene erfindungsgemäße Glaskeramiken hergestellt. Sie hatten die in der Tabelle I angegebenen chemischen Zusammensetzungen.

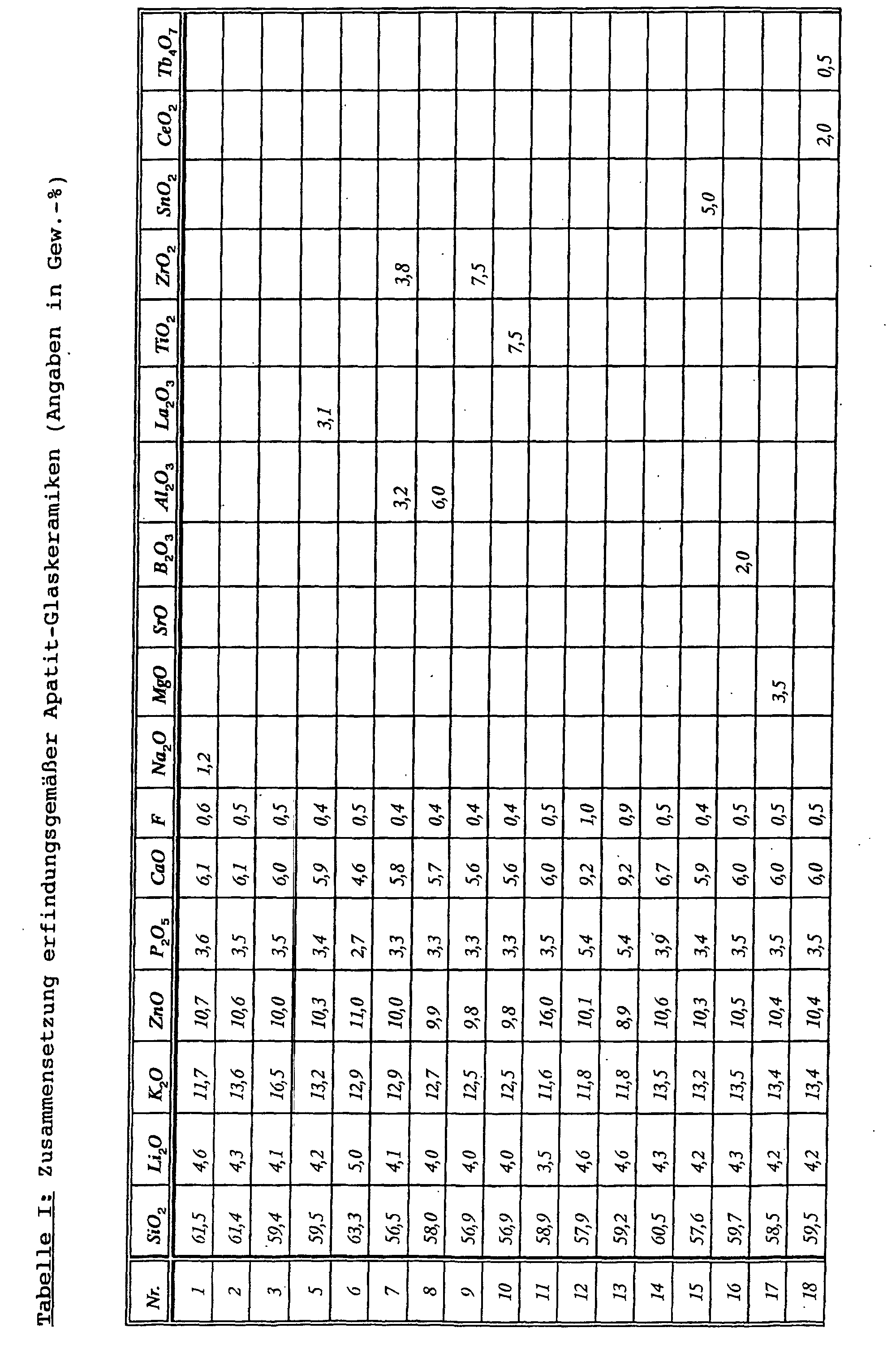

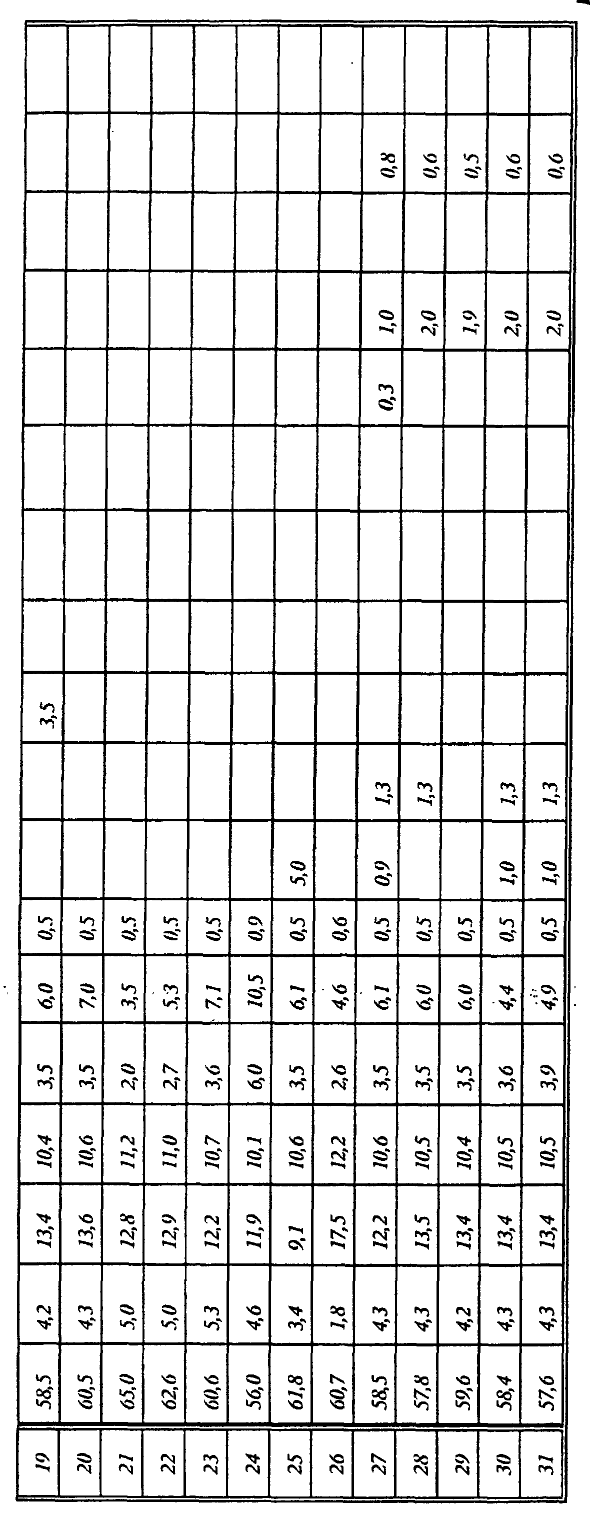

Zu ihrer Herstellung wurde jeweils ein entsprechendes Gemenge von geeigneten Oxiden, Carbonaten und Fluoriden in einem Platin/Rhodium-Tiegel bei einer Temperatur von 1500°C bis 1550°C während einer Homogenisierungszeit von 1 h zu einem Ausgangsglas erschmolzen. Die Glasschmelze wurde in Wasser abgeschreckt, und das gebildete Granulat des Ausgangsglases wurde getrocknet und auf eine mittlere Korngröße von weniger als 90 µm, bezogen auf eine Anzahl der Teilchen, aufgemahlen.

Anschließend wurde das Granulat oder das erhaltene Pulver des Ausgangsglases 30 Minuten bis 6 Stunden einer ein- oder mehrstufigen Wärmebehandlung bei mehr als 500°C und bis zu 900°C unterworfen, woraufhin sich die entsprechende Glaskeramik bildete.

Für einige der Glaskeramiken sind in Tabelle II ausgewählte Eigenschaften angegeben, die an Probekörpern aus der jeweiligen Glaskeramik bestimmt worden sind. Weiter finden sich in Tabelle II unter "Thermischer Behandlung" Angaben zu der konkret gewählten Wärmebehandlung des Ausgangsglases.

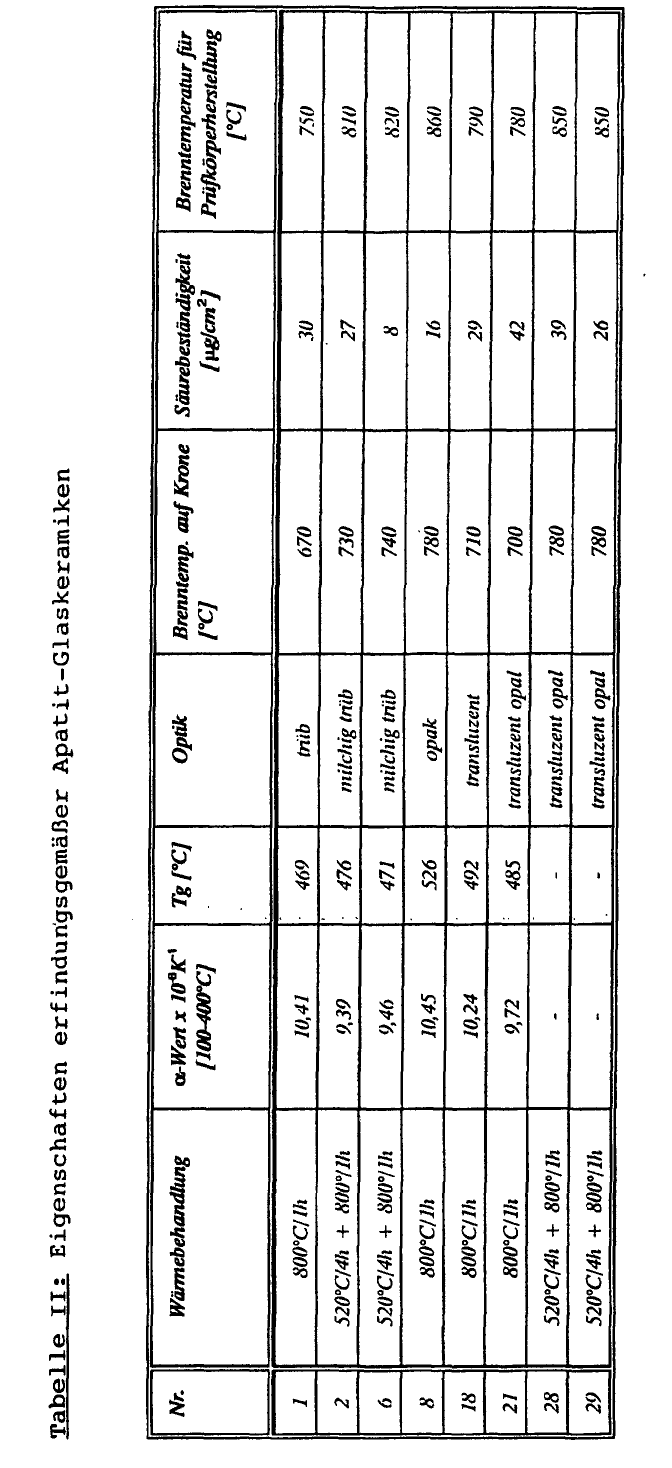

Die "Brenntemp. auf Krone" bezeichnet dabei die Temperatur, bei der die Glaskeramik auf ein Kronengerüst aus glaskeramischem Material aufgesintert werden konnte.

Die Beispiele verdeutlichen wie durch Veränderung der chemischen Zusammensetzung Glaskeramiken mit unterschiedlichen Eigenschaften erhalten werden können.

### Bestimmung des Ausdehungskoeffizienten α

Zur Messung des linearen thermischen Ausdehnungskoeffizienten a wurde aus Pulver der jeweiligen Glaskeramik ein stäbchenförmiger Grünkörper hergestellt, der in einem Vakuumbrennofen mit einer Aufheizrate von 60°C/min und einer Haltezeit von 1 Minute bei der jeweils angegebenen Brenntemperatur für Prüfkörperherstellung gesintert wurde. Anschließend wurde ein Glanzbrand ohne Vakuum bei einer um 20°C höheren Endtemperatur und einer Haltezeit von 1 Minute durchgeführt. An dem erhaltenen Probekörper wurde der lineare thermische Ausdehnungskoeffizient im Temperaturbereich von 100 bis 400°C bestimmt.

### Bestimmung der Säurebeständigkeit

Die Säurebeständigkeit ist ein Maß für die chemische Beständigkeit gerade von im Dentalbereich eingesetzten Glaskeramiken, da diese in der Mundhöhle permanent der Einwirkung von sauren Substanzen ausgesetzt sind.

Die Säurebeständigkeit wurde gemäß der ISO-Vorschrift 6872:1995 bestimmt. Dazu wurden zunächst Probeplättchen mit einem Durchmesser von 12 mm und einer Dicke von 1 mm durch Zusammensintern von Glaskeramik-Pulver mit einer mittleren Teilchengröße von 90 µm hergestellt. Das Pulver wurde 1 Minute lang auf der Sintertemperatur gehalten. Dann wurden die Probeplättchen 16 Stunden mit 4 Vol.-%iger wäßriger Essigsäure bei 80°C behandelt, und es wurde schließlich der eingetretene Masseverlust bezogen auf die Plättchenoberfläche als Maß für die Säurebeständigkeit bestimmt.

Die Beispiele verdeutlichen wie durch Veränderung der chemischen Zusammensetzung Glaskeramiken mit unterschiedlichen Eigenschaften erhalten werden können.

### Beispiel 32

Dieses Beispiel beschreibt den Einsatz einer Mischung der erfindungsgemäßen Apatit-Glaskeramik nach Beispiel 29 zusammen mit einem Kalium-Zink-Silicat-Glas als Beschichtungsmaterial für keramische Suprastrukturen und somit dessen Einsetzbarkeit bei der Herstellung von vollkeramischen Dentalprodukten.

Das verwendete Kalium-Zink-Silicat-Glas hatte die Zusammensetzung (in Gew.-%):
SiO2 65,2 Li₂O 4,2; K₂O 14,8; ZnO 12,8; MgO 0,6; ZrO₂ 1,9; CeO₂ 0,5

Zu Herstellung dieses Kalium-Zink-Silicat-Glases wurde ein entsprechendes Gemenge von Ausgangsstoffen in einem Platin/Rhodium-Tiegel bei einer Temperatur von 1550°C bis 1600°C während einer Homogenisierungszeit von 1 bis 1,5 Stunden erschmolzen. Die Glasschmelze wurde in Wasser abgeschreckt, und das gebildete Granulat des Glases wurde getrocknet und auf eine mittlere Korngröße von weniger als 90 µm aufgemahlen.

Zum Erhalt eines Beschichtungsmaterials, bei dem Sintertemperatur und Ausdehnungskoeffizienten geeignet eingestellt sind, wurden 50 Gew.-% der erfindungsgemäßen Apatitglaskeramik mit 50 Gew.-% des Glases in Form von Pulvern mit einer mittleren Teilchengröße von weniger als 90µm gemischt. Diese Mischung wurde zu einem stäbchenförmigen Grünkörper in einem Vakuumofen bei einer Aufheizgeschwindigkeit von 60°C/min und einer Haltezeit von 1 min bei 840°C gesintert. Für die so erhaltene Probe wurde ein thermischer Ausdehnungskoeffizient von 9,96 x 10⁻⁶K⁻¹, gemessen im Temperaturbereich von 100°C bis 400°C, bestimmt.

Damit konnte diese Mischung zum Aufsintern auf eine sehr transluzente Lithiumdisilicat-Glaskeramik mit einem thermischen Ausdehnungskoeffizienten von 10,6 x 10⁻⁶K⁻¹ verwendet werden. Dabei zeigt sich, daß das Aufsintern der Mischung bereits bei einer Temperatur von lediglich 730°C möglich war. Insgesamt konnten somit vollkeramische Dentalprodukte, wie Kronen oder Brücken, hergestellt werden, die sich durch einen ausgezeichneten Verbund der einzelnen Schichten, ein ästhetisches Aussehen und gute chemische Stabilität auszeichnen.

### Beispiel 33

### Herstellung einer Verblendschale

Es wurde aus einer Lithiumdisilicat-Glaskeramik durch Verpressen im viskosen Zustand eine Verblendschale für einen mittleren oberen Schneidezahn mit einer Schichtdicke von max. 0,5 mm hergestellt. Nach dem Heißpressen wurde die Schichtstärke durch mechanische Nachbearbeitung mit einem Diamantwerkzeug auf max. 0,25 mm reduziert. Danach wurde die Verblendschale in einer wässrigen Lösung von 0,5 Vol.-% HF und 3 Vol.-% H₂SO₄ während 10 Minuten im Ultraschallbad oberflächlich gereinigt und dann mit Al₂O₃ bei einem Strahldruck von 1,5 bar sandgestrahlt. Danach wurde ein Dentalmaterial aufgesintert, und zwar eine Mischung aus der erfindungsgemäßen Glaskeramik 2 und einem Kalium-Zink-Silicat-Glas. Das verwendete Kalium-Zink-Silicat-Glas hatte die Zusammensetzung (in Gew.-%):
SiO₂: 64,0; Li₂O: 4,0; K₂O: 12,6; ZnO: 12,4; Al₂O₃: 3,2; ZrO₂ 3,8.

Zu Herstellung dieses Kalium-Zink-Silicat-Glases wurde ein entsprechendes Gemenge von Ausgangsstoffen in einem Platin/Rhodium-Tiegel bei einer Temperatur von 1550°C bis 1600°C während einer Homogenisierungszeit von 1 bis 1,5 Stunden erschmolzen. Die Glasschmelze wurde in Wasser abgeschreckt, und das gebildete Granulat des Glases wurde getrocknet und auf eine mittlere Korngröße von weniger als 90 µm aufgemahlen.

Zum Erhalt eines Beschichtungsmaterials, bei dem Sintertemperatur und Ausdehnungskoeffizient geeignet eingestellt sind, wurden 50 Gew.-% der erfindungsgemäßen Apatit-Glaskeramik mit 50 Gew.-% des Kalium-Zink-Silicat-Glases in Form von Pulvern mit einer mittleren Teilchengröße von weniger als 90 µm gemischt. Der thermische Ausdehnungskoeffizient dieses Dentalmaterials betrug 9,4 × 10⁻¹⁰K⁻¹. Die Sintertemperatur lag bei 750°C und wurde bei der Beschichtung der Verblendschale während 1 Minute gehalten. Insgesamt wurden 5 Brände mit Materialauftrag bei 750°C bis zur Fertigstellung der Verblendschale durchgeführt. Der abschließende Glanzbrand wurde bei 740°C ohne Vakuum durchgeführt, um einen oberflächlichen Eigenglanz zu erzielen. Die eine Dentalrestauration darstellende Verblendschale zeigte einen sehr homogenen Schichtverbund. Es bildeten sich keine Risse oder Sprünge, was bei so dünnen Wandstärken nicht selbstverständlich ist. Die Verblendschale zeigte weiter eine sehr gute Transluzenz, was bei dieser Form von Dentalrestauration eine überaus wichtige Eigenschaft ist.

### Beispiel 34

### Herstellung einer 3-gliedrigen Frontzahnbrücke

Es wurde aus einer Lithiumdisilikat-Glaskeramik durch Verpressen im viskosen Zustand ein Frontzahnbrückengerüst mit einem Zwischenglied hergestellt. Die kleinste Wandstärke betrug ca. 0,5 mm. Nach dem Heißpressen wurde das Gerüst mit einer wässrigen Lösung von 0,5 Vol.-% HF und 3 Vol.-% H₂SO₄ während 10 Minuten im Ultraschallbad oberflächlich gereinigt und anschließend mit Al₂O₃ bei einem Strahldruck von 1,5 bar sandgestrahlt. Danach wurde ein Dentalmaterial aufgesintert, welches aus der erfindungsgemäßen Apatit-Glaskeramik 28 und einem Kalium-Zink-Silicat-Glas bestand. Das verwendete Kalium-Zink-Silicat-Glas hatte die Zusammensetzung (in Gew.-%):
SiO₂: 65,2; Li₂O: 4,2; K₂O: 14,8; ZnO: 12,8; MgO: 0,6; ZrO₂: 1,9; CeO₂: 0,5.

Zu Herstellung dieses Kalium-Zink-Silicat-Glases wurde ein entsprechendes Gemenge von Ausgangsstoffen in einem Platin/Rhodium-Tiegel bei einer Temperatur von 1550°C bis 1600°C während einer Homogenisierungszeit von 1 bis 1,5 Stunden erschmolzen. Die Glasschmelze wurde in Wasser abgeschreckt, und das gebildete Granulat des Glases wurde getrocknet und auf eine mittlere Korngröße von weniger als 90 µm aufgemahlen.

Zum Erhalt eines Beschichtungsmaterials, bei dem Sintertemperatur und Ausdehnungskoeffizient geeignet eingestellt sind, wurden 50 Gew.-% der erfindungsgemäßen Apatitglaskeramik mit 50 Gew.-% des Kalium-Zink-Silicat-Glases in Form von Pulvern mit einer mittleren Teilchengröße von weniger als 90 µm gemischt. Der thermische Ausdehnungskoeffizient dieses Dentalmaterials betrug 10,0 × 10⁻⁶K⁻¹. Die Sintertemperatur lag bei 730°C und wurde bei der Beschichtung des Gerüstes jeweils 1 Minute lang gehalten. Insgesamt wurden 5 Brände mit Materialauftrag bei 730°C bis zur Fertigstellung der Frontzahnbrücke durchgeführt. Der abschließende Glanzbrand wurde bei 720°C ohne Vakuum durchgeführt, um einen oberflächlichen Eigenglanz zu erzielen. Die erhaltene dreigliedrige Frontzahnbrücke zeigte einen homogenen Verbund zwischen Lithiumdisilikatgerüst und Sintermaterial. Es bildeten sich aufgrund des abgestimmten Wärmeausdehnungskoeffizienten, der niedrigen Sintertemperatur und der chemischen Verträglichkeit zwischen den einzelnen Komponenten keine Risse oder Sprünge in der Brücke.

## Patentansprüche

1. Tiefsinternde Apatit-Glaskeramik, **dadurch gekennzeichnet, daß** sie die folgenden Komponenten enthält:
| Komponente | Gew.-% |
|---|---|
| SiO₂ | 56,0 bis 65,0 |
| Li₂O | 1,8 bis 5,3 |
| K₂O | 9,0 bis 17,5 |
| ZnO | 8,9 bis 16,0 |
| CaO | 3,5 bis 10,5 |
| P₂O₅ | 2,0 bis 6,0 |
| F | 0,4 bis 1,0. |
und die Hauptkristallphase durch Apatit-Kristalle gebildet ist.

2. Apatit-Glaskeramik nach Anspruch 1, **dadurch gekennzeichnet, daß** sie mindestens eine der folgenden Komponenten enthält:
| Komponente | Gew.-% |
|---|---|
| Na₂O | 0 bis 5,0 |
| MgO | 0 bis 3,5 |
| SrO | 0 bis 3,5 |
| Al₂O₃ | 0 bis 6,0 |
| B₂O₃ | 0 bis 2 , 0 |
| La₂O₃ | 0 bis 3, 0 |
| ZrO₂ | 0 bis 7,5 |
| TiO₂ | . 0 bis 7 , 5 |
| CeO₂ | 0 bis 2,0 |
| SnO₂ | 0 bis 5,0 |
| Tb₄O₇ | 0 bis 0,5. |

3. Apatit-Glaskeramik nach Anspruch 1 oder 2, **dadurch gekennzeichnet, daß** die Mengen der Komponenten, sofern nicht anders angegeben, unabhängig voneinander wie folgt sind:
| Komponente | Gew.-% |
|---|---|
| SiO₂ | 56,0 bis 64,0 |
| Li₂O | 2,0 bis 5,0 |
| K₂O | 9,5 bis 16,0 |
| ZnO | 8,9 bis 15,0 |
| CaO | 4,0 bis 10,0 |
| P₂O₅ | 2,0 bis 5,0 |
| F | 0,4 bis 0,9 |
| Na₂O | 0 bis 4 , 0 |
| MgO | 0 bis 3,0 |
| SrO | 0 bis 3,0 |
| Al₂O₃ | 0 bis 5,0 |
| B₂O₃ | 0 bis 1,8 |
| La₂O₃ | 0 bis 2,5 |
| ZrO₂ | 0 bis 6,0 |
| TiO₂ | 0 bis 6,0 |
| CeO₂ | 0 bis 1,8 |
| SnO₂ | 0 bis 4,0 |
| Tb₄O₇ | 0 bis 0,4. |

4. Apatit-Glaskeramik nach einem der Ansprüche 1 bis 3, **dadurch gekennzeichnet, daß** die Mengen der Komponenten unabhängig voneinander wie folgt sind:
| Komponente | Gew.-% |
|---|---|
| SiO₂ | 56,0 bis 63,0 |
| Li₂O | 2,5 bis 5, 0 |
| K₂O | 10,0 bis 15,0 |
| ZnO | 8,9 bis 14,0 |
| CaO | 4,0 bis 9,0 |
| P₂O₅ | 2,5 bis 5,0 |
| F | 0,4 bis 0,8 |
| Na₂O | 0 bis 3,0 |
| MgO | 0 bis 2,5 |
| SrO | 0 bis 2,5 |
| Al₂O₃ | 0 bis 4,0 |
| B₂O₃ | 0 bis 1 , 5 |
| La₂O₃ | 0 bis 2,0 |
| ZrO₂ | 0 bis 5,0 |
| TiO₂ | 0 bis 5,0 |
| CeO₂ | 0 bis 1,5 |
| SnO₂ | 0 bis 3,0 |
| Tb₄O₇ | 0 bis 0, 3 . |

5. Apatit-Glaskeramik nach einem der Ansprüche 1 bis 4, **dadurch gekennzeichnet, daß** sie eine Sintertemperatur von weniger als 800°C aufweist.

6. Apatit-Glaskeramik nach einem der Ansprüche 1 bis 5, **dadurch gekennzeichnet, daß** sie eine Sintertemperatur von 670 bis 780°C aufweist.

7. Apatit-Glaskeramik nach einem der Ansprüche 1 bis 6, **dadurch gekennzeichnet, daß** die Apatit-Kristalle nadelförmig sind.

8. Apatit-Glaskeramik nach einem der Ansprüche 1 bis 7, **dadurch gekennzeichnet, daß** die Apatitkristalle in ihrer größten Ausdehnung kleiner als 10 µm sind.

9. Apatit-Glaskeramik nach einem der Ansprüche 1 bis 8, **dadurch gekennzeichnet, daß** sie einen linearen thermischen Ausdehnungskoeffizienten von 9,3 bis 10,8 × 10⁻⁶K⁻¹, gemessen im Temperaturbereich von 100 °C bis 400 °C, hat.

10. Verfahren zur Herstellung der Apatit-Glaskeramik gemäß einem der Ansprüche 1 bis 9, **dadurch gekennzeichnet, daß**
a) ein Ausgangsglas, welches die Komponenten gemäß einem der Ansprüche 1 bis 4 enthält, bei Temperaturen von 1200°C bis 1650°C erschmolzen wird,
b) die erhaltene Glasschmelze unter Bildung eines Glasgranulates in Wasser eingegossen wird,
c) das Glasgranulat gegebenenfalls zu einem Glaspulver mit einer mittleren Korngröße von 1 bis 450 µm, bezogen auf die Teilchenzahl, zerkleinert wird, und
d) das Glasgranulat oder das Glaspulver einer thermischen Behandlung bei mehr als 500°C und bis zu 900°C für eine Dauer von 30 Minuten bis 6 Stunden unterzogen wird.

11. Dentalmaterial, **dadurch gekennzeichnet, daß** es die Apatit-Glaskeramik gemäß einem der Ansprüche 1 bis 9 enthält.

12. Dentalmaterial nach Anspruch 11, **dadurch gekennzeichnet, daß** es zusätzlich ein Kalium-Zink-Silicat-Glas enthält.

13. Dentalmaterial nach Anspruch 12, **dadurch gekennzeichnet, daß** das Kalium-Zink-Silicat-Glas die folgenden Komponenten enthält:
| Komponente | Gew.-% |
|---|---|
| SiO₂ | 60,0 bis 72,0 |
| Li₂O | 1,0 bis 5,0 |
| K₂O | 10,0 bis 23,0 |
| ZnO | 8,5 bis 20,0. |

14. Dentalmaterial nach einem der Ansprüche 11 bis 13, **dadurch gekennzeichnet, daß** es eine Sintertemperatur von weniger als 800°C aufweist.

15. Verwendung des Dentalmaterials gemäß einem der Ansprüche 11 bis 14 zur Beschichtung eines Substrates und insbesondere zur Beschichtung einer dentalen Restauration.

16. Verwendung nach Anspruch 15, wobei ein Substrat auf Basis von keramischem oder glaskeramischem Werkstoff, insbesondere Lithiumdisilicat-Glaskeramik, eingesetzt wird.

17. Verwendung nach Anspruch 16, wobei die Lithiumdisilicat-Glaskeramik die folgenden Komponenten enthält:
| Komponente | Gew.-% |
|---|---|
| SiO₂ | 57,0 bis 80,0 |
| Al₂O₃ | 0 bis 5,0 |
| La₂O₃ | 0,1 bis 6,0 |
| MgO | 0 bis 5,0 |
| ZnO | 0 bis 8,0 |
| Li₂O | 11,0 bis 19,0 |
| P₂O₅ | 0 bis 11,0 |
wobei
(a) Al₂O₃ + La₂O₃ 0,1 bis 7,0 Gew.-% und
(b) MgO + ZnO 0,1 bis 9,0 Gew.-%
ausmachen.

18. Verwendung nach einem der Ansprüche 15 bis 17, wobei das Dentalmaterial auf das Substrat aufgebracht und bei Temperaturen von weniger als 800°C gesintert wird.

19. Geformtes Dentalprodukt, **dadurch gekennzeichnet, daß** es die Apatit-Glaskeramik gemäß einem der Ansprüche 1 bis 9 oder das Dentalmaterial gemäß einem der Ansprüche 11 bis 14 enthält.

20. Geformtes Dentalprodukt nach Anspruch 19, **dadurch gekennzeichnet, daß** es eine dentale Restauration ist.

21. Geformtes Dentalprodukt nach Anspruch 19 oder 20, **dadurch gekennzeichnet, daß** es einen Kern auf Basis von keramischem oder glaskeramischem Werkstoff und eine darauf aufgebrachte Beschichtung aus der Apatit-Glaskeramik oder dem Dentalmaterial aufweist.

22. Geformtes Dentalprodukt nach Anspruch 21, **dadurch gekennzeichnet, daß** der glaskeramische Werkstoff eine Lithiumdisilicat-Glaskeramik ist.

## Claims

1. Low-sintering apatite glass-ceramic, **characterized**
**in that** it comprises the following components:
| Component | wt.-% |
|---|---|
| SiO₂ | 56.0 to 65.0 |
| Li₂O | 1.8 to 5.3 |
| K₂O | 9.0 to 17.5 |
| ZnO | 8.9 to 16.0 |
| CaO | 3.5 to 10.5 |
| P₂O₅ | 2.0 to 6.0 |
| F | 0.4 to 1.0 |
and the main crystal phase is formed by apatite crystals.

2. Apatite glass-ceramic according to Claim 1, **characterized in that** it comprises at least one of the following components:
| Component | wt.-% |
|---|---|
| Na₂O | 0 to 5.0 |
| MgO | 0 to 3.5 |
| SrO | 0 to 3.5 |
| Al₂O₃ | 0 to 6.0 |
| B₂O₃ | 0 to 2.0 |
| La₂O₃ | 0 to 3.0 |
| ZrO₂ | 0 to 7.5 |
| TiO₂ | 0 to 7.5 |
| CeO₂ | 0 to 2.0 |
| SnO₂ | 0 to 5.0 |
| Tb₄O₇ | 0 to 0.5. |

3. Apatite glass-ceramic according to Claim 1 or 2, **characterized in that** the amounts of the components, unless stated otherwise, are, independently of one another, as follows:
| Component | wt.-% |
|---|---|
| SiO₂ | 56.0 to 64.0 |
| Li₂O | 2.0 to 5.0 |
| K₂O | 9.5 to 16.0 |
| ZnO | 8.9 to 15.0 |
| CaO | 4.0 to 10.0 |
| P₂O₅ | 2.0 to 5.0 |
| F | 0.4 to 0.9 |
| Na₂O | 0 to 4.0 |
| MgO | 0 to 3.0 |
| SrO | 0 to 3.0 |
| Al₂O₃ | 0 to 5.0 |
| B₂O₃ | 0 to 1.8 |
| La₂O₃ | 0 to 2.5 |
| ZrO₂ | 0 to 6.0 |
| TiO₂ | 0 to 6.0 |
| CeO₂ | 0 to 1.8 |
| SnO₂ | 0 to 4.0 |
| Tb₄O₇ | 0 to 0.4. |

4. Apatite glass-ceramic according to one of Claims 1 to 3, **characterized in that** the amounts of the components are, independently of one another, as follows:
| Component | wt.-% |
|---|---|
| SiO₂ | 56.0 to 63.0 |
| Li₂O | 2.0 to 5.0 |
| K₂O | 10.0 to 15.0 |
| ZnO | 8.9 to 14.0 |
| CaO | 4.0 to 9.0 |
| P₂O₅ | 2.5 to 5.0 |
| F | 0.4 to 0.8 |
| Na₂O | 0 to 3.0 |
| MgO | 0 to 2.5 |
| SrO | 0 to 2.5 |
| Al₂O₃ | 0 to 4.0 |
| B₂O₃ | 0 to 1.5 |
| La₂O₃ | 0 to 2.0 |
| ZrO₂ | 0 to 5.0 |
| TiO₂ | 0 to 5.0 |
| CeO₂ | 0 to 1.5 |
| SnO₂ | 0 to 3.0 |
| Tb₄O₇ | 0 to 0.3. |

5. Apatite glass-ceramic according to one of Claims 1 to 4, **characterized in that** it has a sintering temperature of less than 800°C.

6. Apatite glass-ceramic according to one of Claims 1 to 5, **characterized in that** it has a sintering temperature of from 670 to 780°C.

7. Apatite glass-ceramic according to one of Claims 1 to 6, **characterized in that** the apatite crystals are needle-shaped.

8. Apatite glass-ceramic according to one of Claims 1 to 7, **characterized in that** the apatite crystals are smaller than 10 µm in their greatest dimension.

9. Apatite glass-ceramic according to one of Claims 1 to 8, **characterized in that** it has a linear thermal expansion coefficient of from 9.3 to 10.8 × 10⁻⁶K⁻¹, measured in the temperature range from 100°C to 400°C.

10. Process for the preparation of the apatite glass-ceramic according to one of Claims 1 to 9, **characterized in that**
a) a starting glass which comprises the components according to one of Claims 1 to 4 is melted at temperatures of from 1200°C to 1650°C,
b) the glass melt obtained is poured into water with formation of glass granules,
c) the glass granules are optionally comminuted to give a glass powder having a mean particle size of from 1 to 450 µm, based on the number of particles, and
d) the glass granules or the glass powder is subjected to thermal treatment at more than 500°C and up to 900°C for a period of from 30 minutes to 6 hours.

11. Dental material, **characterized in that** it comprises the apatite glass-ceramic according to one of Claims 1 to 9.

12. Dental material according to Claim 11, **characterized in that** it additionally comprises a potassium-zinc-silicate glass.

13. Dental material according to Claim 12, **characterized in that** the potassium-zinc-silicate glass comprises the following components:
| Component | wt.-% |
|---|---|
| SiO₂ | 60.0 to 72.0 |
| Li₂O | 1.0 to 5.0 |
| K₂O | 10.0 to 23.0 |
| ZnO | 8.5 to 20.0. |

14. Dental material according to one of Claims 11 to 13, **characterized in that** it has a sintering temperature of less than 800°C.

15. Use of the dental material according to one of Claims 11 to 14 for coating a substrate and in particular for coating a dental restoration.

16. Use according to Claim 15, where a substrate based on ceramic or glass-ceramic material, in particular lithium disilicate glass-ceramic, is employed.

17. Use according to Claim 16, where the lithium disilicate glass-ceramic comprises the following components:
| Component | wt.-% |
|---|---|
| SiO₂ | 57.0 to 80.0 |
| Al₂O₃ | 0 to 5.0 |
| La₂O₃ | 0.1 to 6.0 |
| MgO | 0 to 5.0 |
| ZnO | 0 to 8.0 |
| Li₂O | 11.0 to 19.0 |
| P₂O₅ | 0 to 11.0 |
where
(a) Al₂O₃ + La₂O₃ make up 0.1 to 7.0 wt.-% and
(b) MgO + ZnO make up 0.1 to 9.0 wt.-%

18. Use according to one of Claims 15 to 17, where the dental material is applied to the substrate and sintered at temperatures of less than 800°C.

19. Shaped dental product, **characterized in that** it comprises the apatite glass-ceramic according to one of Claims 1 to 9 or the dental material according to one of Claims 11 to 14.

20. Shaped dental product according to Claim 19, **characterized in that** it is a dental restoration.

21. Shaped dental product according to Claim 19 or 20, **characterized in that** it has a core based on ceramic or glass-ceramic material and a coating of the apatite glass-ceramic or the dental material applied thereto.

22. Shaped dental product according to Claim 21, **characterized in that** the glass-ceramic material is a lithium disilicate glass-ceramic.

## Revendications

1. Vitrocéramique d'apatite à frittage intense, **caractérisée en ce qu'**elle contient les composants suivants :
| Composants | % en poids |
|---|---|
| SiO₂ | 56,0 à 65,0 |
| Li₂O | 1,8 à 5,3 |
| K₂O | 9,0 à 17,5 |
| ZnO | 8,9 à 16,0 |
| CaO | 3,5 à 10,5 |
| P₂O₅ | 2,0 à 6,0 |
| F | 0,4 à 1,0 |
et **en ce que** la phase cristalline principale est formée de cristaux d'apatite.

2. Vitrocéramique d'apatite selon la revendication 1, **caractérisée en ce qu'**elle contient au moins l'un des composants suivants :
| Composants | % en poids |
|---|---|
| Na₂O | 0 à 5,0 |
| MgO | 0 à 3,5 |
| SrO | 0 à 3,5 |
| Al₂O₃ | 0 à 6,0 |
| B₂O₃ | 0 à 2,0 |
| La₂O₃ | 0 à 3,0 |
| ZrO₂ | 0 à 7,5 |
| TiO₂ | 0 à 7,5 |
| CeO₂ | 0 à 2,0 |
| SnO₂ | 0 à 5,0 |
| Tb₄O₇ | 0 à 0, 5 |

3. Vitrocéramique d'apatite selon la revendication 1 ou 2, **caractérisée en ce que** les quantités des composants, sauf indication contraire, sont les suivantes indépendamment les unes des autres :
| Composants | % en poids |
|---|---|
| SiO₂ | 56,0 à 64,0 |
| LiO₂ | 2,0 à 5,0 |
| K₂O | 9,5 à 16, 0 |
| ZnO | 8,9 à 15,0 |
| CaO | 4,0 à 10,0 |
| P₂O₅ | 2, 0 à 5, 0 |
| F | 0,4 à 0,9 |
| Na₂O | 0 à 4,0 |
| MgO | 0 à 3,0 |
| SrO | 0 à 3,0 |
| Al₂O₃ | 0 à 5,0 |
| B₂O₃ | 0 à 1,8 |
| La₂O₃ | 0 à 2, 5 |
| ZrO₂ | 0 à 6,0 |
| TiO₂ | 0 à 6,0 |
| CeO₂ | 0 à 1,8 |
| SnO₂ | 0 à 4, 0 |
| Tb₄O₇ | 0 à 0,4 |

4. Vitrocéramique d'apatite selon l'une quelconque des revendications 1 à 3, **caractérisée en ce que** les quantités des composants sont les suivantes indépendamment les unes des autres :
| Composants | % en poids |
|---|---|
| SiO₂ | 56,0 à 63,0 |
| LiO₂ | 2,5 à 5,0 |
| K₂O | 10,0 à 15, 0 |
| ZnO | 8,9 à 14,0 |
| CaO | 4,0 à 9,0 |
| P₂O₅ | 2, 5 à 5, 0 |
| F | 0,4 à 0,8 |
| Na₂O | 0 à 3,0 |
| MgO | 0 à 2,5 |
| SrO | 0 à 2,5 |
| Al₂O₃ | 0 à 4,0 |
| B₂O₃ | 0 à 1,5 |
| La₂O₃ | 0 à 2,0 |
| ZrO₂ | 0 à 5,0 |
| TiO₂ | 0 à 5,0 |
| CeO₂ | 0 à 1,5 |
| SnO₂ | 0 à 3,0 |
| Tb₄O₇ | 0 à 0,3 |

5. Vitrocéramique d'apatite selon l'une quelconque des revendications 1 à 4, **caractérisée en ce qu'**elle présente une température de frittage inférieure à 800°C.

6. Vitrocéramique d'apatite selon l'une quelconque des revendications 1 à 5, **caractérisée en ce qu'**elle présente une température de frittage de 670 à 780°C.

7. Vitrocéramique d'apatite selon l'une quelconque des revendications 1 à 6, **caractérisée en ce que** les cristaux d'apatite sont de forme aciculaire.

8. Vitrocéramique d'apatite selon l'une quelconque des revendications 1 à 7, **caractérisée en ce que** les cristaux d'apatite ont une taille inférieure à 10 micromètres dans leur étendue maximale.

9. Vitrocéramique d'apatite selon l'une quelconque des revendications 1 à 8, **caractérisée en ce qu'**elle a un coefficient de dilatation thermique linéaire de 9,3 à 10,8 x 10⁻⁶K⁻¹, mesuré dans la plage de températures de 100°C à 400°C.

10. Procédé de fabrication de la vitrocéramique d'apatite selon l'une quelconque des revendications 1 à 9, **caractérisé en ce que** :
a) un verre de départ, qui contient les composants selon l'une quelconque des revendications 1 à 4, est fondu à des températures de 1200°C à 1650°C;
b) la masse fondue de verre obtenue est coulée dans de l'eau pour former un granulé de verre;
c) le granulé de verre est éventuellement broyé en une poudre de verre ayant une granulométrie moyenne de 1 à 450 micromètres en fonction du nombre de particules; et
d) le granulé de verre ou la poudre de verre est soumis à un traitement thermique à plus de 500°C et jusqu'à 900°C sur une période de 30 minutes à 6 heures.

11. Matériau dentaire, **caractérisé en ce qu'**il contient la vitrocéramique d'apatite selon l'une quelconque des revendications 1 à 9.

12. Matériau dentaire selon la revendication 11, **caractérisé en ce qu'**il contient en outre un verre de potassium-zinc-silicate.

13. Matériau dentaire selon la revendication 12, **caractérisé en ce que** le verre de potassium-zinc-silicate contient les composants suivants :
| Composants | % en poids |
|---|---|
| SiO₂ | 60,0 à 72,0 |
| LiO₂ | 1,0 à 5,0 |
| K₂O | 10,0 à 23,0 |
| ZnO | 8,5 à 20,0 |

14. Matériau dentaire selon l'une quelconque des revendications 11 à 13, **caractérisé en ce qu'**il présente une température de frittage de moins de 800°C.

15. Utilisation du matériau dentaire selon l'une quelconque des revendications 11 à 14 pour le revêtement d'un substrat, en particulier, pour le revêtement d'un appareil de restauration dentaire.

16. Utilisation selon la revendication 15, dans laquelle on utilise un substrat à base de matériau céramique ou vitrocéramique, en particulier une vitrocéramique de disilicate de lithium.

17. Utilisation selon la revendication 16, dans laquelle la vitrocéramique de disilicate de lithium contient les composants suivants :
| Composants | % en poids |
|---|---|
| SiO₂ | 57,0 à 80,0 |
| Al₂O₃ | 0 à 5,0 |
| La₂O₃ | 0,1 à 6, 0 |
| MgO | 0 à 5,0 |
| ZnO | 0 à 8,0 |
| Li₂O | 11,0 à 19,0 |
| P₂O₅ | 0 à 11, 0 |
dans lesquels :
(a) Al₂O₃ + La₂O₃ constituent 0,1 à 7,0% en poids; et
(b) MgO + ZnO constituent 0,1 à 9,0% en poids

18. Utilisation selon l'une quelconque des revendications 15 à 17, dans laquelle le matériau dentaire est appliqué sur le substrat et est fritté à des températures de moins de 800°C.

19. Produit dentaire moulé, **caractérisé en ce qu'**il contient la vitrocéramique d'apatite selon l'une quelconque des revendications 1 à 9 ou le matériau dentaire selon l'une quelconque des revendications 11 à 14.

20. Produit dentaire moulé selon la revendication 19, **caractérisé en ce qu'**il s'agit d'un appareil de restauration dentaire.

21. Produit dentaire moulé selon la revendication 19 ou 20, **caractérisé en qu**'il présente un noyau à base de matériau céramique ou vitrocéramique et un revêtement en vitrocéramique d'apatite ou en matériau dentaire qui est appliqué par-dessus.

22. Produit dentaire moulé selon la revendication 21, **caractérisé en ce que** le matériau vitrocéramique est une vitrocéramique de disilicate de lithium.
